# EUROPEAN PATENT APPLICATION

(11) **EP 2 003 197 A2**
(43) Date of publication of application: **17.12.2008**
(21) Application number: 06841754.2
(22) Date of filing: 22.12.2006
(51) Int. Cl.: C12N 9/24, C12N 9/04, A61K 38/47, A61K 38/44

(54) **SYNERGISTIC EFFECT BETWEEN A CYANOGENIC SYSTEM AND ANOTHER OXIDATIVE INDUCER FOR THE TREATMENT OF TUMOURS**

(30) Priority: 23.12.2005 ES 200503173
(71) Applicant: Universidad Autónoma de Madrid, 28049 Madrid (ES)
(72) Inventor: IZQUIERDO ROJO, Marta, E-28049 Cantoblanco (Madrid) (ES); GARCIA-ESCUDERO BARRERAS, Vega, E-28049 Cantoblanco (Madrid) (ES); GARGINI, Ricardo, E-28049 Cantoblanco (Madrid) (ES)
(74) Representative: Carpintero Lopez, Francisco
(86) International application number: PCT/ES2006/000702
(87) International publication number: WO 2007/074191

(57) **Abstract**

The invention relates to a system capable of causing the death of tumor cells by activating caspase-independent apoptosis, comprising:
I. a cyanogenic system comprising the enzymatic activity exerted by linamarase on linamarin and an oxidative stress inducing system comprising the oxidative activity by the glucose oxidase enzyme combined in a single composition or
II. a cyanogenic system comprising the enzymatic activity exerted by linamarase on linamarin and an oxidative stress inducing system comprising the oxidative activity exerted by the glucose oxidase enzyme present in independent compositions.

## Description

The invention relates to a composition and method able to kill tumor cells, comprising the synergistic activity established between the cyanogenic and oxidative stress inductor systems.

### Prior State of the Art

The linamarase-linamarin system is based on the use of a gene of plant origin (linamarase, *lis*) encoding a β-glucosidase which is able to transform the innocuous linamarin substrate (lin, 2-hydroxy-isobutyronitrile-β-D-glucopyranoside) to produce acetone cyanohydrin and glucose (Cortés, *et al*.. 1998, Hughes, *et al* 1992; Cortés, *et al.,* 2002). Acetone cyanohydrin is unstable at pH exceeding 6 and temperatures greater than 30ºC, spontaneously being transformed into acetone and cyanide (Selmar, *et al.,* 1987). The linamarase gene is introduced in the target cells by means of retroviral vectors, (e.g., murine leukemia virus, MLV, derivatives) adenoviral vectors (e.g., Ad5 derivatives) or non-viral vectors (plasmid transfection). When this gene is expressed in mammal cells the linamarase is transported outside the cell where it meets with its linamarin substrate and cyanide is produced (Cortés, *et al.,* 2002). Cyanide has the ability to diffuse freely through cell membranes (Wisler, *et al.,* 1991) and affects not only the cells producing linamarase but also the surrounding cells, therefore the system is associated to a collateral effect increasing the therapeutic potential. Cyanide has the ability to bind to and inactivate the cytochrome c oxidase enzyme, blocking the electron transport chain of oxidative phosphorylation, negatively affecting the mitochondrial respiration function. This increases the production of reactive oxygen species (ROS) in the mitochondrion while at the same time blocking the production of energy in the form of adenosine triphosphate (ATP) through oxidative phosphorylation.

The rapid depletion of intracellular levels of ATP by blockage of oxidative phosphorylation induces cell death by necrosis. However, the oxidative stress caused by this blockage is limited, therefore in the invention, this therapy has been combined with a local treatment with glucose oxidase (GO) catalyzing the conversion of glucose into gluconic acid, generating hydrogen peroxide (H₂O₂). The increase of H₂O₂ promotes the generation of reactive oxygen species (ROS), the excess of which negatively affects tumor cells.

Until now there has been diverse literature describing the use of the linamarase/linamarin system in gene therapy. In addition, the cytotoxic effect of glucose oxidase has already been described and there are therapeutic combinations against malignant tumors based on enzyme-antibody conjugates in which one of the enzymes is glucose oxidase and the other one is peroxidase.

However, based on the previous description and knowledge and as can be seen in the description below, the technique of the invention is based on the fact that the combination of both systems, *lis*/lin and GO, has not previously been described. Based on said synergistic activity, the therapeutic power of the linamarase/linamarin system is enhanced because the oxidative stress levels achieved in tumor cells considerably increases when the glucose oxidase system is used in combination with the linamarase/linamarin system.

Therefore, in one aspect of the invention, a vector is used to introduce the linamarase gene and the glucose oxidase enzyme in the tumor cell. This produces in the area surrounding the tumor cell two systems, a cyanogenic system and another oxidative stress inducing system which, by acting synergistically, will produce the early death of tumor cells by activating caspase-independent apoptosis.

The combination of both systems, *lis*/lin and GO, causes a clear synergistic reaction resulting in the production of ROS as a consequence, on one hand, of the blockage of oxidative phosphorylation caused by cyanide and, on the other hand, of the H₂O₂ produced by glucose oxidase. Therefore, since oxidative stress is a cell death initiating signal, it is used as a strategy for treating cancer.

The importance of this technique is based on the fact that the combination of both systems amply exceeds the effect observed by the mere addition of responses when both systems acted separately, causing remarkable synergism in the production of oxidative stress. Combining both systems involves an evident time advance in the death of tumor cells in relation to the death observed in said cells when they were treated only by means of the *lis*/lin system or only by the addition of GO.

This combination additionally causes the switching of the type of death from necrosis to caspase-independent apoptosis but by means of a different mechanism from those known until now because it is independent of AIF and PARP (Poly[ADP-Ribose] polymerase).

The invention further provides several possibilities with regard to the inclusion of the glucose oxidase system. In addition, the glucose oxidase gene could be introduced in the tumor cells through a viral or non-viral vector. Another possible improvement would be to inoculate the purified enzyme by means of controlled release systems or by means of the use of the recombinant protein bound to antibodies directed against tumor antigens.

Most traditional therapies, such as chemotherapy and radiotherapy, fail because tumor cells accumulate mutations making them resistant to said treatments, especially to those treatments promoting death by caspase-dependent apoptosis, such as mutations in the genes corresponding to caspases, p53, etc. It is therefore necessary to develop new therapies which are able to overcome the resistance acquired by tumor cells, for example systems which generate a type of caspase-independent death such as that described in the invention. The extraordinary increase of oxidative stress caused by the combined *lis*/lin/GO therapy system in the cells, it is a strategy that is able to cause rapid mitochondrial fragmentation, which promotes the release of proapoptotic factors converging in inducing cell death by a caspase-independent apoptosis process. This system counteracts the possible known mechanisms of resistance of tumor cells to death. Furthermore, since one of the aspects of this invention is based on a local injection of adenovirus, the invention allows infecting the tumor cells which are actively dividing as well as the vast majority that are quiescent. This also includes infecting the tumor stem cells which are believed to be the tumor initiators and responsible for metastasis and relapses, the main cause of mortality due to cancer. Additionally, due to the fact that the production of cyanide and peroxide is mainly extracellular, this invention compensates for the low rates of *in vivo* infection. Likewise, by affecting both the cells producing linamarase and those surrounding it, it prevents any tumor cell from escaping therapy.

### DESCRIPTION OF THE INVENTION

### Brief Description of the Invention

A first aspect of the invention relates to a system able to cause the death of tumor cells by activating caspase-independent apoptosis, comprising:
a) a cyanogenic system comprising the enzymatic activity exerted by linamarase on linamarin and an oxidative stress inducing system comprising the oxidative activity exerted by the glucose oxidase enzyme combined in a single composition, (hereinafter single composition) or
b) a cyanogenic system comprising the enzymatic activity exerted by linamarase on linamarin and an oxidative stress inducing system comprising the oxidative activity exerted by the glucose oxidase enzyme present in independent compositions (hereinafter compositions of the invention).

Within the invention, single composition is understood as that composition comprising at least the indispensable elements (in the form of vectors, proteins or others) of the cyanogenic system and of the oxidative stress system and being able to cause the death of tumor cells by activating caspase-independent apoptosis.

Within the invention, compositions of the invention are understood as at least two compositions in which at least one of them comprises at least one indispensable element (in the form of a vector, protein or others) of the cyanogenic system and the other one comprises at least one indispensable element (in the form of a vector, protein or others) of the oxidative stress system and the combination of which causes the death of tumor cells by activating caspase-independent apoptosis.

Within the invention, when indistinctly referring to the single composition or the compositions of the invention, it refers to the composition of the invention.

In a preferred aspect of the invention, the cyanogenic system of the composition of the invention comprises the linamarase-linamarin system, i.e., the enzymatic activity exerted by linamarase on linamarin, which acts as a substrate of the reaction.

In another aspect of the invention, the oxidative stress inducing system of the composition of the invention comprises the activity of the glucose oxidase enzyme, i.e., the oxidative activity exerted by the glucose oxidase enzyme on the glucose acting as a substrate.

In a second aspect of the invention, the cyanogenic system of the composition of the invention comprises the linamarase-linamarin system, and the oxidative stress inducing system of the composition of the invention comprises the glucose oxidase enzyme.

In a third aspect of the invention, the composition of the invention comprises the linamarase gene and the glucose oxidase gene combined in a single viral or non-viral vector.

In a fourth aspect of the invention, the composition of the invention comprises the linamarase gene and the glucose oxidase gene in independent viral or non-viral vectors.

In a fifth aspect of the invention, the composition of the invention comprises the linamarase gene, introduced in a viral or non-viral vector, and the purified glucose oxidase protein or analogues, fragments or derivatives of said protein.

In a preferred aspect, the composition of the invention comprises the glucose oxidase gene, introduced in a viral or non-viral vector, and the purified linamarase protein or analogues, fragments or derivatives of said protein.

In a more preferred aspect of the invention, the composition of the invention comprises the glucose oxidase protein and the linamarase protein or analogues, fragments or derivatives of said proteins.

In a preferred embodiment of the invention, the composition of the invention comprises the purified glucose oxidase protein and/or the purified linamarase protein as well as analogues, fragments or derivatives of said proteins and a controlled release system for releasing said proteins.

In a more preferred embodiment of the invention, the composition of the invention comprises the purified glucose oxidase recombinant protein and/or the purified linamarase recombinant protein as well as analogues, fragments or derivatives of said proteins bound to antibodies directed against tumor antigens.

A sixth aspect of the invention comprises a viral or non-viral vector able to cause the death of tumor cells by activating caspase-independent apoptosis, comprising a cyanogenic system and an oxidative stress inducing system.

A preferred aspect of the invention comprises a viral or non-viral vector able to cause the death of tumor cells by activating caspase-independent apoptosis, comprising the combination of the linamarase and glucose oxidase genes or any modification thereof, either naturally or by genetic engineering, in a single vector.

In yet another aspect of the invention, it comprises at least two viral or non-viral vectors comprising the linamarase and glucose oxidase genes or any modification thereof, either naturally or by genetic engineering, in independent vectors, the combination of which is able to cause the death of tumor cells by activating caspase-independent apoptosis.

Another embodiment of the invention comprises an adenoviral and/or retroviral vector comprising the linamarase and/or glucose oxidase genes.

In a seventh aspect of the invention, it comprises the use of the vector/vectors of the invention for preparing a pharmaceutical composition intended for treating tumors.

In a preferred aspect of the invention, it relates to the composition of the invention for its use in therapy.

In yet a more preferred aspect of the invention, it relates to the use of the composition of the invention for preparing a pharmaceutical composition for treating tumors.

In another embodiment of the invention, it relates to any of the previously mentioned pharmaceutical compositions and a pharmaceutically acceptable carrier.

In an eighth aspect of the invention, it comprises but is not limited to a pharmaceutical composition intended for treating breast cancer, lung cancer, head and neck cancer, pancreatic cancer, prostate cancer, colon cancer, melanomas, osteosarcoma, adenocarcinoma, leukemia or glioblastoma.

A ninth aspect of the invention comprises an *in vitro* or *in vivo* method (hereinafter method of the invention) able to cause the death of tumor cells by activating caspase-independent apoptosis, comprising the combination of two systems: a cyanogenic system and an oxidative stress inducing system.

In a preferred aspect of the invention, the cyanogenic system of the method of the invention comprises the linamarase-linamarin system.

In a more preferred aspect, the oxidative stress inducing system of the method of the invention comprises the activity of the glucose oxidase enzyme.

In yet a more preferred aspect of the invention, the oxidative stress inducing system of the method of the invention comprises the activity of the glucose oxidase enzyme and the cyanogenic system of the method of the invention comprises the linamarase-linamarin system.

In yet another aspect of the invention, any of the compositions of the invention are used in the embodiment of the method of the invention.

In a preferred aspect of the invention, any of the pharmaceutical compositions or vectors of the invention are used for the embodiment of the method of the invention.

Within the invention, analogues, derivatives or fragments of the linamarase or glucose oxidase proteins are understood as those which are able to produce the synergistic effect claimed in the invention.

Throughout the descriptions and the claims of the specification, the word "comprises" and variations thereof do not mean to exclude other aspects of the invention which will be obvious for a person skilled in the art in view of the description.

The detailed explanation of the embodiments, examples and the following figures are provided by way of illustration and do not mean to limit the invention.

### Description of the Figures

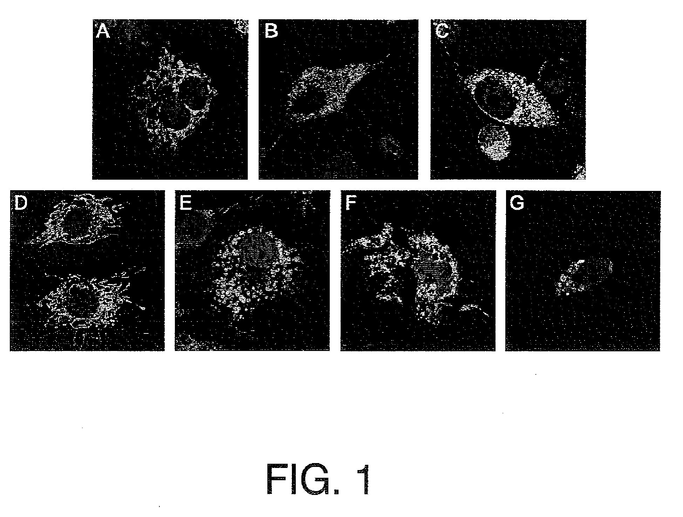

Figure 1 shows confocal microscope images of cells having red mitochondria by transfection of the plasmid pdsRed2-mito, and a blue nucleus by staining with To-Pro-3. Images representing the mitochondrial pattern of the untreated control cells (A), cells treated with linamarin (500 µg/ml) at 48 hours (B) and 72 hours (C) after beginning treatment, cells treated with glucose oxidase (5 mEU/ml) (D) and cells treated with the combined therapy of glucose oxidase (5 mEU/ml) and linamarin (500 µg/ml) at 24 hours (E and F) or at 48 hours (G) after treatment are shown.

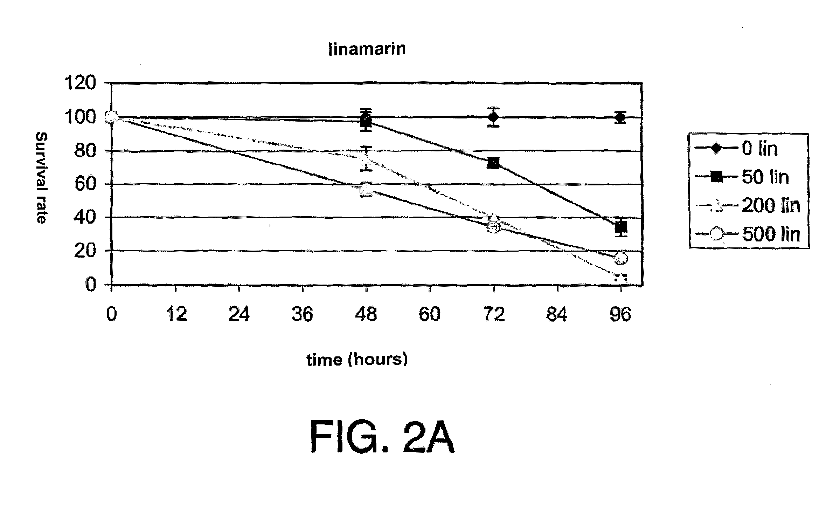

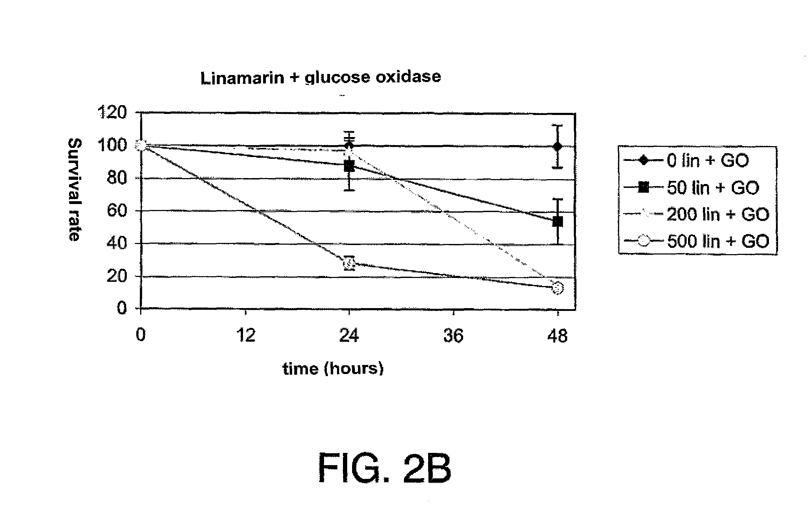

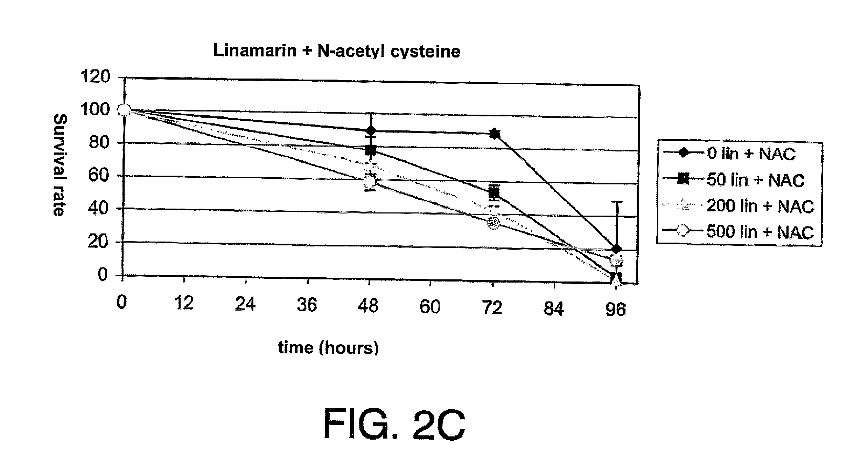

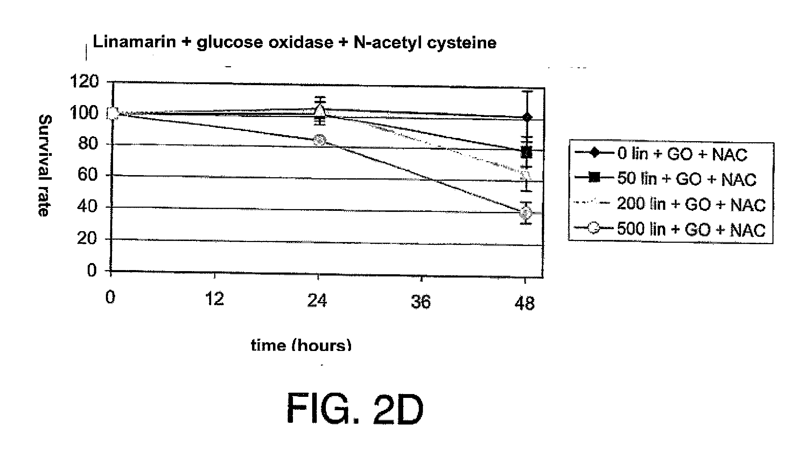

Figures 2(A) and 2(B) show the analysis of cell survival/death depending on the mitochondrial activity of W&W*lis* cells. The survival rate over time of the cells treated with linamarin (A) and with linamarin and 5 mEU/ml of glucose oxidase (B) are shown. Figures 2(C) and 2(D) show the cell survival when an antioxidant (10 mM NAC) is added in the treatment.

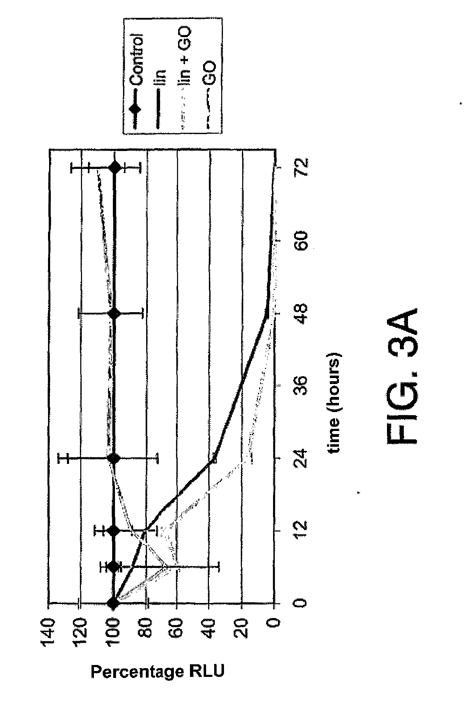

Figure 3(A) shows the study of the reduction of intracellular ATP levels. The estimation was done over time in W&W*lis* cells treated with linamarin (500 µg/ml) and/or glucose oxidase (5 mEU/ml). The values show the mean ± the standard deviation of the percentage of relative light units (RLU) of two independent samples for each point with regard to the value obtained from the untreated cells. Figure 2(B) shows the production of extracellular hydrogen peroxide.

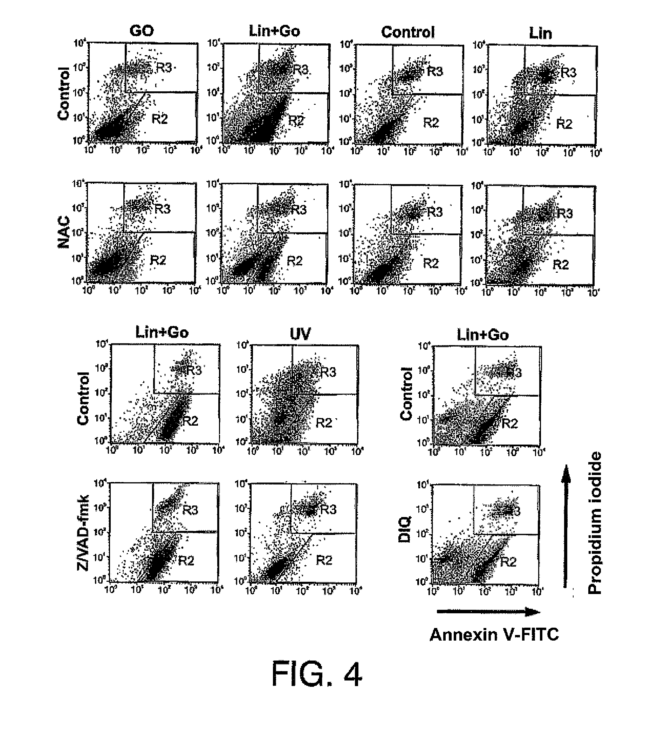

Figure 4 shows the characterization of the type of death of the system in W&W*lis* cells by flow cytometry, labeling with annexin V-FITC and propidium iodide.

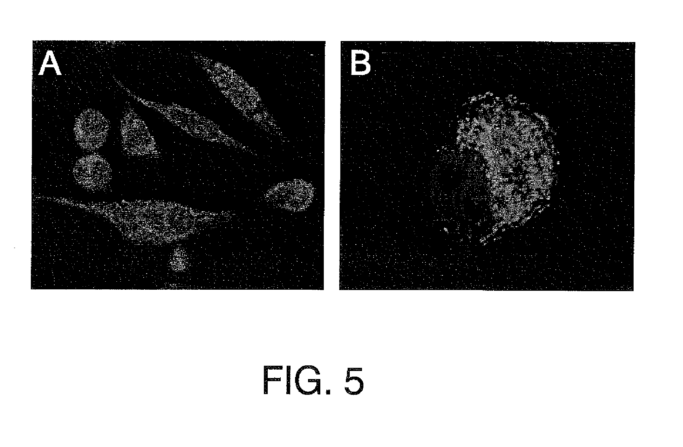

Figure 5 shows the AIF location study by immunofluorescence in W&W*lis* cells. The nuclei are labeled with To-Pro-3. The cells were treated with 5 mEU/ml of glucose oxidase (A) or glucose oxidase and 500 µg/ml of linamarin (B).

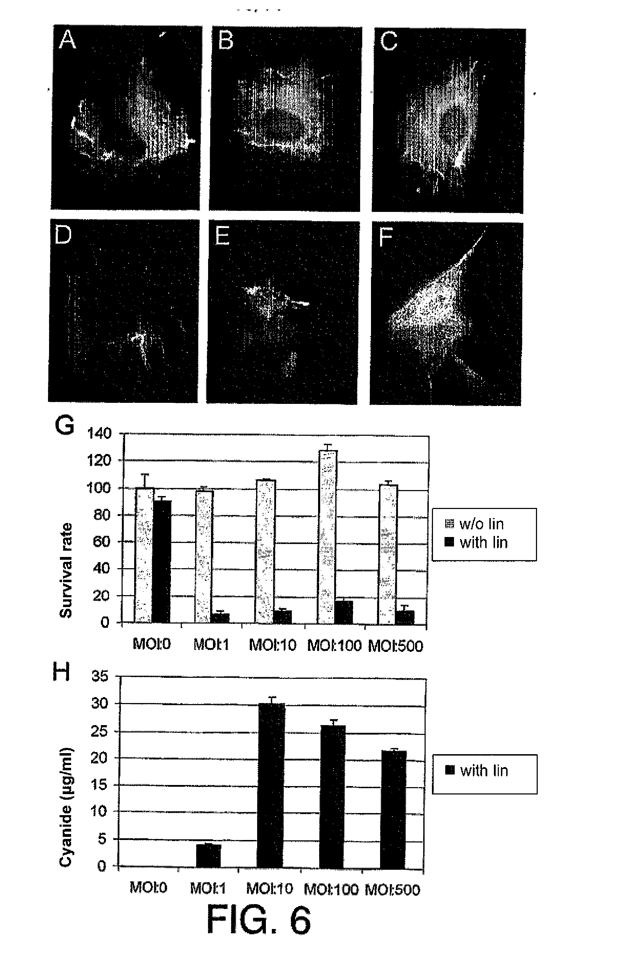

Figure 6 shows the study of the infection of patient explant cells by adeno*lis*. Detection of linamarase by immunofluorescence in the cells of patients GB-LP-1 (A), GB-LP-2 (B), GB-LP-3 (C), GB-LP- 4 (D), GB-LP-5 (E) and GB-RC-1 (F), infected with adeno*lis*. Study of the survival rate by MTT of the GB-LP-1 cells against infection with adeno*lis* in the presence or absence of 500 µg/ml of linamarin at different multiplicities of infection (MOI) (G). Production of cyanide in µg/ml of the GB-LP-1 cells infected at different MOI in the presence of 500 µg/ml of linamarin (H). The graphs (G and H) depict the means ± the standard deviation of 3 independent samples.

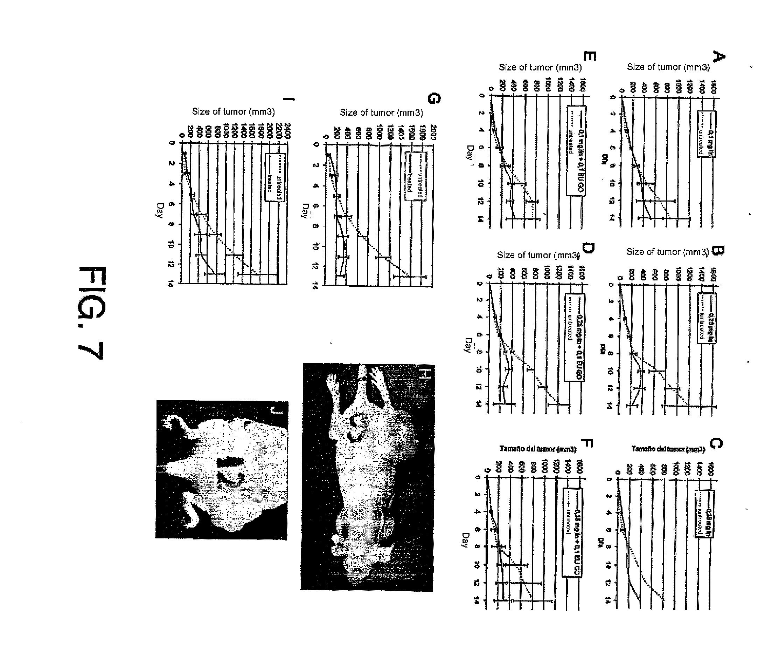

Figure 7 shows the study of the therapeutic action of the system in immunodeficient mice. The mice were inoculated with W&W*lis* cells (A-H) or with W&W cells (I and J) in both flanks, one of which was treated with 0.1 mg/g (A), 0.25 mg/g (B) and 0.35 mg/g of linamarin (C); or with the combined therapy with 0.1 mEU/ml of GO and 0.1 mg/g (D), 0.25 mg/g (E) and 0.35 mg/g of linamarin (F). Study of the efficacy of the treatment of W&W*lis* tumors with 0.25 mg/g of lin and 0.1 mEU/ml of GO (G) and representative image of one of the mice of the group (H). Study of the combined therapy using adeno*lis* in mice with W&W tumors treated with 0.25 mg/g of lin and 0.1 mEU/ml of GO (I) and representative image of one of the mice of the group (J). The graphs show the mean ± the standard errors of the volumes expressed in mm³ of 4 mice (A, C, E and F), 6 mice (B and D), 8 mice (G) and 10 mice (I) over time.

### Detailed Description of the Invention

The invention relates, among other aspects, to the composition and method intended for treating tumor cells, comprising the synergistic activity established between two systems: cyanogenic and oxidative stress inducing systems.

The cyanogenic system comprises the enzymatic activity exerted by linamarase on linamarin, which acts as a substrate of the reaction. The oxidative stress inducing system comprises the oxidative activity exerted by the glucose oxidase enzyme on the glucose acting as a substrate.

For the purpose of studying the mitochondrial dynamics occurring as a result of the oxidative stress caused by the activity of the claimed system, W&W*lis* cells were stably transfected with the plasmid pdsRed2-mito (Clontech) containing the gene of the one red fluorescent protein carried by a transport signal peptide to the mitochondrion. This allowed viewing the mitochondria of cells treated with linamarin and glucose oxidase by fluorescence microscopy. It could thus be verified that while the untreated cells showed a filamentous mitochondrial pattern (Figure 1 A), in the cells treated with linamarin this matrix is disrupted, acquiring a dotted pattern (Figures 1B and 1C). This effect starts to be observed 48 hours after treatment with linamarin (Figure 1 B) and becomes more pronounced after 72 hours, a slight swelling of the fragments (Figure 1 C) being observed. Treatment with glucose oxidase alone did not cause any structural change in most of the mitochondria (Figure 1 D). However, when both linamarin and glucose oxidase treatments are combined, a dotted pattern of swollen mitochondria was observed after 24 hours (Figure 1 E) in which a high percentage of cells is observed with a fragmented nuclear morphology characteristic of apoptosis (Figure 1 F). After 48 hours the few cells which remain adhered to the substrate showed pronounced swelling of the mitochondria (Figure 1 G).

The analysis of cell survival by MTT (based on evaluating the formation of formazan crystals from tetrazolium by live cells) showed that in the treatment with linamarin, the viability of the cells begins to slightly decrease after 48 hours in proportion to the concentration of linamarin (Figure 2A). However, it is not until 96 hours that a drastic reduction of the survival was observed and only at high concentrations of linamarin (200 to 500 µg/ml). When combined therapy with linamarin and glucose oxidase was carried out, cell viability began to decrease after 24 hours at high concentrations of linamarin (500 µg/ml) and after 48 hours survival is virtually zero (Figure 2B). This demonstrates that the combined therapy causes an advance of death of approximately 48 hours increasing the aggressiveness of the system, which translates into greater therapeutic efficacy.

The success of linamarase/linamarin/glucose oxidase therapy is due to the fact that the combination of both systems causes a synergism in the production of oxidative stress. To confirm this hypothesis, the behavior of the cells in therapy is observed when 10 mM N-acetyl cysteine (NAC) is added in the medium, although the same results were also found at other concentrations, which is a potent antioxidant. It can be observed in the cell viability assay by MTT that while the addition of NAC did not cause any effect on the linamarase/linamarin therapy (Figure 2C), in the case of the *lis*/lin/GO combination a pronounced inhibition of death does occur (Figure 2D). Similar results were obtained in death with annexin V-FITC/propidium iodide (Figure 4, Table 3).

The levels of intracellular energy in the form of ATP decrease when a blockage of the mitochondrial electron transport chain occurs since it is the essential source of obtaining energy of the cells. When intracellular ATP levels are analyzed, it is observed that the addition of glucose oxidase caused a moderate and transitory reduction of ATP after 6 hours (Figure 3A) which completely remits after 24 hours, after which time the levels coincide with the control cells. This effect is due to the fact that the maximum H₂O₂ production by glucose oxidase occurs at short times because the cells have the ability of detoxifying the peroxide and due to the fact that glucose oxidase is inactivated over time by the serum proteins. The addition of linamarin caused a progressive and rapid depletion of ATP which becomes almost complete after 48 hours as a result of the blockage of mitochondrial respiration at the level of cytochrome c by the cyanide produced. In the combination of both treatments it was observed that an initial reduction of the levels occurs after 6 hours due to the production of peroxide by glucose oxidase, after 12 hours the ATP levels drop in parallel to the treatment with linamarin, although the levels are somewhat lower due to the synergism of both treatments. These results demonstrated that both death due to linamarin, occurring between 72 and 96 hours, and death due to the combination of linamarin and glucose oxidase, occurring between 24 and 48 hours, are triggered by an mechanism independent of energy because ATP levels are very low in these time intervals.

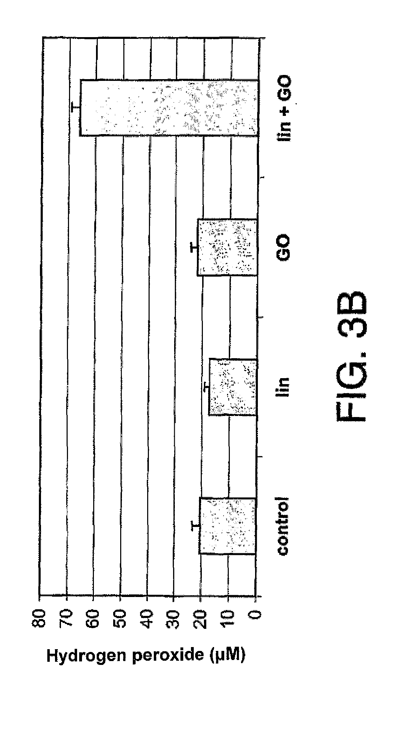

The H₂O₂ levels (Figure 3B) produced extracellularly in the combined therapy were further studied in the invention. To do so, the amount of H₂O₂ present in the culture medium of W&W*lis* cells at 48 hours in a treatment with 0.5 mg/ml of linamarin, in the presence of 5 mEU/ml of glucose oxidase or in the combination of both treatments, was analyzed although positive results were also obtained using other concentrations. It was observed that while treatment with linamarin or with glucose oxidase did not cause significant variations in the extracellular H₂O₂ concentration with regard to the control (approximately 20 µM), the combination of both treatments produced a synergistic increase in the production of peroxide at the extracellular level of 65.9 µM. This data confirms the hypothesis that the success of the combined therapy is due to the synergism in the production of oxidative stress.

In addition, the combined *lis*/lin/GO system unexpectedly transformed the pattern of death by necrosis characteristic of cyanide (*lis*/lin) into ATP-independent apoptosis which further advanced cell death about 48 hours, therefore the therapy is more effective. Since most apoptotic mechanisms, such as the formation of apoptosome, are ATP-dependent, it is important to determine the proteins that are involved in the death in the present model. First the involvement of caspases was analyzed, to which end an analysis of death by flow cytometry, labeling with annexin V-FITC and propidium iodide in the presence of the pan-caspase inhibitor Z-VAD-fmk (100 µM) (Table 4 and Figure 4) was performed. It was observed that caspase inhibition does not cause any effect on the combined *lis*/lin/GO therapy, therefore it can be concluded that the apoptosis is caspase-independent. Cells irradiated by UV (12.5 J/m²), which causes a typical death by caspase-dependent apoptosis, were used as control. It was verified that the addition of Z-VAD-fmk (100 µM) caused the complete inhibition of the UV radiation-induced apoptosis both by analysis of the DNA content (Table 1) and by labeling with annexin V-FITC and propidium iodide (Figure 4).

The contribution of the PARP-1 (Poly[ADP-Ribose] polymerase) protein in the combined system was then studied. This protein senses damages to the DNA that is in the nucleus and after its activation, it translocates to the mitochondrion in which it activates apoptotic proteins such as AIF (Hong, *et al.,* 2004). This pathway is one of the possible mechanisms of apoptosis generated by ROS. A specific PARP inhibitor, 1,5-isoquinolinediol (DIQ) (Table 5), was used to study the contribution of this protein in the combined system of the invention. It was observed that apoptosis caused by *lis*/lin/GO is not mediated by the activation pathway of AIF, which depends on PARP, since it is not inhibited by DIQ.

Nevertheless, the activation of AIF could be caused by a mechanism that is PARP-1-independent (Cregan, 2004). AIF is a protein residing in the mitochondrion and when activated, it translocates to the nucleus in which it activates apoptosis. The possible translocation of AIF was analyzed by immunofluorescence with antibodies specific against AIF of W&W*lis* cells treated with the therapy. It was observed that the control cells treated with GO show a typically mitochondrial AIF pattern (Figure 5A) and when they are treated with linamarin and GO (Figure 5B) fragmentation of the mitochondrial filaments occurs but AIF remains in the mitochondrion without translocating to the nucleus. This demonstrates that the *lis*/lin/GO system causes apoptosis which is independent of the death mediated by AIF.

The annexin V-FITC and propidium iodide assay was performed in parallel by flow cytometry. The pattern of death of the system was analyzed when adding 5 mEU/ml of glucose oxidase and the combined therapy (5 mEU/ml of GO and 500 µg/ml of linamarin) at 48 hours, followed by the control of untreated cells and of the cells incubated with 500 µg/ml of linamarin at 96 hours. Furthermore, another group of cells was subjected to the same conditions except 10 mM of N-acetyl cysteine (NAC) were added. The cells treated with the combined therapy were subsequently studied at 48 hours in the absence and presence of 100 µM of Z-VAD-fmk, followed by the control of treatment of the cells with UV in the same conditions. Finally, the cells treated with the combined therapy were shown at 48 hours in the absence and presence of 300 µM of 1,5-isoquinolinediol (DIQ), observing that the pattern of death is not modified when PARP-1 is inhibited (Figure 4).

Based on these results it could be concluded that the death triggered by the combined *lis*/lin/GO therapy occurs due to a mechanism of apoptosis which is caspase-, PARP-1- and AIF-independent.

The applicability of the linamarase/linamarin system in explants obtained from six glioblastomas in patients was evaluated to study the potential of the use of the therapy of the invention in patients. First the susceptibility of these explants to infection by the adenovirus carrying the linamarase gene (adeno*lis*) was studied. To that end, these cultures were infected with adeno*lis* and the expression of linamarase was analyzed by immunofluorescence with antibodies specific against linamarase (Figure 6 A-F). It was verified that 5 out of 6 explants were efficiently infected by adeno*lis*, a very active expression of linamarin occurring, which even caused filamentous aggregates in the cells in virtually 100% of the infected cells at a multiplicity of infection (MOI) of 100. Only one of the explants (GB-LP-5, Figure 6 E) showed less than 5% of cells which expressed at MOI: 100. This could be because these cells showed a low expression of CAR (Coxsackie-adenovirus receptor), which is the adenovirus receptor.

The behavior of the *lis*/lin therapy in one of the explants (GB-LP-1) was additionally analyzed. These cells were infected with adeno*lis* (MOI: 0, 1, 12, 100 and 500) and the survival and production of cyanide after the addition of 500 µg/ml linamarin (Figures 6G and 6H, respectively) were studied. These cells were efficiently infected by adeno*lis*, which made them very sensitive to treatment with linamarin, even at very low multiplicities of infection (MOI: 1). Additionally, toxicity was not detected by the vector, even at very high multiplicities of infection (MOI: 100 and 500).

The toxicity of glucose oxidase was evaluated in Swiss mice weighing about 20 grams, intraperitoneally and intravenously inoculated with different amounts of the purified enzyme. Doses of 1 and 0.5 EU/g of weight of the animal showed a lethal effect in the first 24 hours post-administration. However, daily doses between 0.25 and 0.1 EU/g of weight showed no harmful effect. This allowed establishing an optimal dose range for GO for the combined therapy in this type of animal.

In addition, a dose range for linamarin was evaluated in immunodeficient (nude) mice weighing 20 grams, inoculated with W&W tumor cells which stably expressed linamarase (W&W*lis*). When the tumor reached an approximate size of 50 mm³, the mice were treated with a daily dose of intratumoral linamarin at different concentrations (0.5; 0.35; 0.25 or 0.10 mg lin/g of weight of the animal). The dose of 0.1 mg lin/g showed no therapeutic effect (n=4) (Figure 7A), however with 0.25 mg lin/g, significant reduction of the growth of the treated tumor was achieved versus the untreated tumor after the tenth day of treatment (p=0.05; n=6) (Figure 7 B). The increase of the linamarin dose not only improved the therapeutic results but it also caused the death of three of the four animals with the daily dose of 0.35 mg lin/g in the fifth day of treatment (Figure 7C) and of all the animals treated with 0.5 mg lin/g between the fourth and fifth day of treatment.

For the purpose of improving these results by increasing the oxidative stress like in cell cultures, 0.1 EU/g of glucose oxidase was introduced with the treatment with linamarin. The results improved substantially in all cases (Figures 7D-7F) and even decreasing the toxicity at high doses of linamarin (0.35 mg lin/g, Figure 7F). The best therapeutic results were obtained with the daily treatment of 0.25 mg lin and 0.1 EU GO/g where the difference between the treated and untreated tumor became significant after the 8^{th} day of treatment (Figure 7E; p=0.05; n=6), even though pharmacologically positive results were also obtained with other concentrations of glucose oxidase. These results allow affirming that said synergistic activity achieves enhancing the therapeutic power of the linamarase/linamarin system and is therefore a more effective pharmacological system.

Another approach carried out for the invention was the use of adenoviruses carrying the linamarase gene (adeno*lis*) in W&W cell tumors induced in immunodeficient mice. 10⁶ cells were subcutaneously inoculated in both flanks in mice (n=18). Part of the animals (n=8) received W&W*lis* cells which already expressed linamarase, while others (n=10) were inoculated with W&W cells and only when the tumor had developed was it locally infected with adeno*lis*. The animals inoculated with W&W*lis* were treated daily with 0.25 mg lin and 0.1 EU GO/g in the largest tumor at the beginning of treatment (approximately 50 mm³) (Figures 7G and 7H). The animals showing W&W tumors were treated with infection cycles with 10⁹ IU of adeno*lis*, followed by a two-day treatment with 0.25 mg lin and 0.1 EU GO/g (Figures 7I and 7J). The progression of the tumors was evaluated every two days. The results showed that there were significant differences (p=00.5) between treated and untreated tumor after the seventh day of treatment in the case of tumors which already expressed the linamarase gene (Figure 7G) and after the eleventh day of treatment in the case of infection by adeno*lis* (Figure 7I), which demonstrated the efficiency of the treatment with adenovirus.

The system was assayed in glioblastomas induced in the flanks of immunodeficient mice and in dogs. Glioblastoma cells were implanted in dogs by means of stereotaxis processes in their brain, thus causing a tumor, which is treated with the claimed process.

Plasmid, retroviral and adenoviral vectors were used in the invention as a means of introducing the linamarase plant gene in the tumor cell. Once said gene was inside the cancerous cell it was expressed, the linamarase enzyme being synthesized. The linamarase enzyme was secreted naturally outside the cell, where it met with its linamarin substrate, and catalyzed the reaction by means of which the linamarin, introduced in the animal by means of injection, was broken down into glucose and acetone cyanohydrin, which spontaneously gave rise to two compounds, acetone and cyanide, the latter being responsible for the death of the tumor cell. Nevertheless, other ways of carrying out the death of tumor cells by activating caspase-independent apoptosis comprising the combination of the cyanogenic and oxidative stress inducing systems would fall within the scope of the invention. Additionally, the doses of Lin and of GO used in the detailed description of the invention and in the tables are set forth only for illustrative purposes and to affirm the potential and efficacy of the invention; nevertheless other therapeutically effective doses would also form part of the scope of the invention.

### EXAMPLES OF THE EMBODIMENT OF THE INVENTION:

### Example 1. Main steps taken to carry out one aspect of the invention.

Verification of the efficacy of therapy in the Wodinsky & Waker cell line, cultured *in vitro,* introducing the linamarase gene by means of the adenovirus vector and the linamarin intratumorally.

### Example 2. Obtaining a cell line which stably expresses linamarase.

The W&W canine glioblastoma cells (Wodinsky, *et al.,* 1969) were transfected with the plasmid pILE (6.9 Kb), which has the CMV (588 bp) promoter, followed by an intron and a polyclone region into which the gene *epolis* (linamarase carrying the extracellular signal of exportation of the human erythropoietin) (1625 bp) was cloned, followed by an IRES (568 bp) and the gene *pac* (resistance to puromycin) (602 bp) and the polyadenylation signal of SV40.

Cationic lipids, Lipofectamine Plus (Invitrogene), were used for the transfection following the commercial company's indications. 2 µg of DNA, 12 µl of Lipofectamine and 8 µl of the reagent Plus were used. Then a stable expression seeding of the linamarase was obtained by selection with 1 µg/ml of puromycin, which shall be referred to as W&W*lis*, and was used for the remaining experiments.

### Example 3. DNA content analysis.

2-5x10⁵ W&W*lis* cells were seeded in 25 cm² flasks. After 24 hours they were treated with linamarin (500 µg/ml) and glucose oxidase (5 mEU/ml). After the time indicated in each assay elapsed, the cells were harvested and washed 2 times with PBS. The cell precipitate was resuspended in 300 µl of PBS at 4ºC. They were subsequently fixed by slowly adding 700 µl of absolute ethanol at -20ºC under stirring. After more than 24 hours, the cells were washed with PBS supplemented with 1% bovine serum albumin and propidium iodide was added at a final concentration of 20 µg/ml, incubating it at room temperature for 1 hour. Data acquisition and analysis was carried out by means of a FACSCalibur (DB Biosciences) flow cytometer with the Cell Quest program.

### Example 4. Infection with adenovirus.

10⁵ cells (W&W and patient explants) were seeded in test tubes with a flat side and screw-on cap (Nunc), or 5x10⁴ cells were seeded on cover slips in 24-well plates and were simultaneously infected with adeno*lis* (Crucell) at different multiplicities of infection (MOI: 0; 0.2; 1; 10; 100 or 500). For the cells seeded in test tubes, the medium was changed after 24 hours adding 0.5 mg/ml of linamarin where appropriate. A cell viability assay was conducted with MTT at 96 hours after the addition of linamarin. 48 hours after infection the cells seeded in the cover slips were processed to perform immunofluorescence to detect linamarase.

### Example 5. Analysis of the structural modifications of the mitochondrion during treatment.

To study the structural modifications of the mitochondrion during therapy, the W&W and W&W*lis* cells were transfected with the plasmid pDsRed2-mito (Clontech, BD Bioscience) containing the gene of a red fluorescent protein which is expressed exclusively in mitochondria. The transfection was performed similarly to the previous section and clones were selected by resistance to neomycin (0.75 and 1.5 mg/ml respectively). The cells from stable expression clones of the protein were seeded in multiwell plates on cover slips (2.5x10⁴) and were treated with linamarin (50, 200 or 500 µg/ml) and glucose oxidase (5 mEU/ml). After the suitable incubation time for each experiment (24, 48 or 72 hours) elapsed, the cells were fixed with 4% paraformaldehyde. The nuclei were stained with To-Pro-3 (Molecular Probes) with a 1/500 dilution for 30 minutes. The structural changes of the mitochondrion were viewed with a Radiance2000 (BioRad) confocal system coupled to an Axiovert S100 TV (Zeiss) inverted microscope, taking 3 consecutive planes at a distance of 0.2 µm in the red and blue filters, which were mixed.

### Example 6. Cell viability analysis by MTT.

The mitochondrial enzymatic activity as indicative of cell viability was determined by means of using tetrazolium salts. In this assay 10⁵ cells/test tube (culture test tubes with a flat side and screw-on cap to prevent the exit of HCN; Nunc) or 10⁴ cells/well of 24-well plates closed with parafilm were seeded. After 24 hours of incubation fresh culture medium supplemented with the concentrations of linamarin (between 50 and 500 µg/ml) and glucose oxidase (5 mEU/ml) necessary for each experiment was added. In the assays in which it applied, the medium was supplemented with N-acetyl cysteine (10 mM), 1,5-isoquinolinediol (300 µM) or Z-VAD-fmk (100 µM). After the time stipulated in each experiment (between 12 and 96 hours), the culture medium was removed and fresh medium with 200 µg/mL of MTT (3-[4,5-dimethylthiazo-2-yl]-2,5-diphenyl tetrazolium bromide) was added. After 1.5 hours of incubation, the medium was removed and 3 ml (for the test tubes) or 1 ml (for the 24-well plates) of dimethyl sulfoxide was added to dissolve the formazan. After 10 minutes, the absorbance of the samples at 540 nm was determined. MTT is a tetrazolium salt soluble in aqueous medium (yellow) which is transformed by the mitochondrial dehydrogenases in formazan, an insoluble compound in aqueous medium (purple).

### Example 7. Immunofluorescences.

The cells were seeded on cover slips in 24-well plates and were treated according to the requirements of each experiment. After the appropriate incubation time for each assay, the cells were fixed with 4% paraformaldehyde for 30 minutes at room temperature or methanol at -20ºC for 10 minutes. The samples were subsequently permeabilized 10 minutes with PBS supplemented with triton X-100 or 0.1% sodium dodecyl sulfate (SDS) and were blocked by incubating for 30 minutes with PBS with 0.1% triton X-100 and with 1% bovine serum albumin, or PBS with 0.01% SDS and a 10% fetal calf serum. They were subsequently incubated for 45 minutes with the primary antibody at room temperature or overnight at 4ºC. The antibodies used were anti-linamarase (1/200, supplied by Monica Hughes) and anti-AIF (1/50, Cell Signaling Technology). The secondary antibody used was anti-rabbit IgG coupled to fluorescein (1/50, Amersham Pharmacia Biotech). The nuclei were subsequently stained, incubating for 30 minutes with a 1/500 dilution of To-Pro-3 (Molecular Probes). The samples were washed 2 times with PBS and one time with distilled H₂O and were mounted on microscope slides using Mowiol-DABCO or Polong Gold Antifade (Invitrogen) mounting medium.

### Example 8. Determination of the extracellular peroxide concentration.

The samples to be analyzed were diluted in 1 ml of 0.1 M phosphate buffer pH 7.4. Then 3.7 EU/ml of peroxidase (Sigma-Aldrich, St. Louis, USA) and 0.1 mg/ml of ortho-dianisidine (Sigma-Aldrich, St. Louis, USA) were added. The samples were incubated at room temperature for 30 minutes and absorbances at 436 nm were determined. The H₂O₂ concentration was obtained by interpolation of the data obtained in a standard line performed with increasing concentrations of H₂O₂.

### Example 9. Measurement of ATP.

10⁵ W&W*lis* cells were seeded in test tubes with a screw-on cap and flat side (Nunc). After 24 hours, the medium was changed, adding linamarin (500 µg/ml) and glucose oxidase (5 mEU/ml) where appropriate. After 4, 8, 12, 24, 48 or 72 hours, the cells were centrifuged and processed following the instructions of the ATP Bioluminescence Assay Kit CLS II, of Roche Applied Science. The luminescence was analyzed with a Monolight 2010 luminometer (Analytical Luminescence Laboratory, San Diego) and expressed in relative light units (RLU).

### Example 10. Analysis of the type of death by flow cytometry (Annexin V-FITC/IP).

10⁵ W&W*lis* cells were seeded in test tubes with a screw-on cap and flat side (Nunc). After 24 hours, different amounts of linamarin (50, 200 or 500 µg/ml) and glucose oxidase (5 mEU/ml) were added in the specified cases. In the appropriate assays, the medium was supplemented with N-acetyl cysteine (10 mM), 1,5-isoquinolinediol (300 µM) or Z-VAD-fmk (100 µM). After the time determined for each assay (30, 48, 72 or 96 hours), the cells were precipitated, washed with phosphate buffered saline (PBS) and trypsinized. They were centrifuged for 5 minutes at 1200 rpm and the cell precipitate was washed first with PBS and then with binding buffer (0.1 M Hepes/NaOH pH 7.4, 1.4 M NaCl and 25 mM CaCl₂). The cells were again precipitated and resuspended in 100 µl of binding buffer supplemented with 5 µl of annexin V-FITC and 2.5 µg/ml of propidium iodide. The samples were incubated for 15 minutes in the dark. Data acquisition and analysis was carried out by means of a FACSCalibur flow cytometer (DB Biosciences) with the Cell Quest program. Early apoptotic cells were considered to be those cells which showed positive labeling only for annexin V-FITC and late apoptosis or necrosis when they showed positive staining for both annexin-V-FITC and propidium iodide.

### Example 11. Obtaining primary cultures of patient glioblastoma explants.

The biopsies of the tumors from experimental animals were maintained in MEM medium with 20% SFT at 4ºC until they were processed. The samples were cut into approximately 1 mm³ pieces in sterility conditions. Then MEM with 20% SFT supplemented with collagenase (106 EU/ml), 0.1 M HEPES buffer pH 7.4, fungizone (0.5 µg/ml) and DNAse (0.02%) was added and incubated for 16 hours at room temperature. Then the large undigested fragments were removed by decantation and the cells were harvested by centrifugation. The cells were seeded in MEM with 20% SFT until forming a culture. Once the culture was formed, after several steps the medium was replaced with DMEM with 10% SFT.

### Example 12. Therapy in a xenotransplant model with immunodeficient mice tumor cells.

Immunodeficient (*nude* strain), athymic mice weighing approximately 20 grams and 2 months old were used. They were inoculated with 1-2x10⁶ cells (W&W or W&W*lis*) subcutaneously in both flanks of the mice by means of an injection with a volume of 50 µl in complete PBS (supplemented with calcium and magnesium ions) with 0.1% glucose. The size of the tumors (pi π/6 x height x width x length) was measured with a calibrator.

A large group of mice (n=8) were inoculated with 1-2x10⁶ W&W*lis* cells as previously described. When the tumors reached a mean size of 30-50 mm³, the tumors of one flank were treated daily with 0.25 mg of linamarin and 0.1 EU of glucose oxidase/g of weight of the animal. When the untreated tumors reached a size of about 2000 mm³ the animals were sacrificed and it was considered the end of treatment. The data was analyzed statistically by Student's t test, considering a level of significance of 5%.

For the treatment with adenoviruses carrying the linamarase gene, when the tumors of the mice inoculated with W&W cells (n=10) reached a size of 30-50%, treatment cycles were given in one of the flanks. On the first day of the cycle, 10⁹ IU of adeno*lis* were inoculated, being distributed throughout the entire tumor with the aid of a syringe. At 24 hours, a two-day treatment was administered with 0.25 mg/g of linamarin supplemented with 0.1 EU/g of glucose oxidase. This cycle was repeated until the untreated tumors reached a size of about 2000 mm³. The animals were sacrificed and it was considered the end of treatment. The data was analyzed statistically by Student's t test, considering a level of significance of 5%.

**Table 1. Study of the DNA content by flow cytometry, labeling with propidium iodide.**

| | 5 mEU/ml GO | | Without GO | |
|---|---|---|---|---|
| | 24 hours | 48 hours | 48 hours | 72 hours |
| 0 µg lin | 1.94 | 1.03 | 1.80 | 1.84 |
| 50 µg lin | 1.86 | 13.12 | 1.95 | 2.77 |
| 200 µg lin | 2.74 | 41.11 | 1.89 | 3.14 |
| 500 µg lin | 3.00 | 50.47 | 3.21 | 4.91 |
| UV (24 hours) | Control= 66.14 | | Z-VAD-fmk= 2.71 | |

**Table 2. Characterization of the type of death of the system**

| lin | | 24 h | 48 h | 48 h | 72 h | 96 h |
|---|---|---|---|---|---|---|
| 0 µg | Apoptosis | 5.98 | 8.34 | 11.57 | 2.52 | 11.47 |
| | Necrosis | 12.27 | 4.50 | 5.65 | 2.83 | 13.79 |
| 50 µg | Apoptosis | 2.93 | 5.66 | 3.71 | 3.74 | 20.83 |
| | Necrosis | 8.18 | 3.20 | 2.96 | 5.28 | 11.33 |
| 200 µg | Apoptosis | 7.48 | 41.70 | 3.84 | 4.62 | 14.31 |
| | Necrosis | 8.78 | 8.16 | 5.68 | 9.34 | 45.15 |
| 500 µg | Apoptosis | 4.89 | 82.52 | 5.17 | 6.25 | 18.99 |
| | Necrosis | 5.55 | 5.76 | 5.54 | 6.91 | 24.98 |
| | | 5 mEU/ml GO | | Without GO | | |

**Table 3. Study of the inhibition of death by the addition of an antioxidant.**

| N-acetyl cysteine (10 mM) | | 48 hours | | 96 hours | |
|---|---|---|---|---|---|
| | | control | NAC | control | NAC |
| 0 µg lin | Apoptosis | 8.34 | 5.98 | 11.47 | 16.17 |
| | Necrosis | 4.50 | 5.42 | 13.79 | 13.46 |
| 50 µg lin | Apoptosis | 5.66 | 5.59 | 20.83 | 6.44 |
| | Necrosis | 3.20 | 7.22 | 11.33 | 51.89 |
| 200 µg lin | Apoptosis | 41.70 | 18.81 | 14.31 | 21.58 |
| | Necrosis | 8.16 | 9.05 | 45.15 | 29.18 |
| 500 µg lin | Apoptosis | 82.52 | 29.97 | 18.99 | 18.53 |
| | Necrosis | 5.76 | 9.15 | 24.98 | 19.89 |
| | | 5 mEU/ml GO | | Without GO | |

**Table 4. Study of the pattern of death bv caspase inhibition.**

| Z-VAD-fmk (100 µM) | | 48 hours | | 72 hours | |
|---|---|---|---|---|---|
| | | control | Z-VAD-fmk | control | Z-VAD-fmk |
| 0 µg lin | Apoptosis | 3.27 | 13.94 | 2.29 | 2.66 |
| | Necrosis | 29.59 | 18.92 | 15.31 | 37.25 |
| 50 µg lin | Apoptosis | 5.98 | 13.35 | 1.36 | 3.48 |
| | Necrosis | 12.87 | 13.31 | 14.72 | 15.01 |
| 200 µg lin | Apoptosis | 64.85 | 66.76 | 3.30 | 2.56 |
| | Necrosis | 10.10 | 8.18 | 30.13 | 13.07 |
| 500 µg lin | Apoptosis | 90.71 | 74.06 | 9.02 | 6.08 |
| | Necrosis | 4.62 | 14.57 | 18.04 | 13.24 |
| | | 5 mEU/ml GO | | Without GO | |

**Table 5. Study of the pattern of death by PARP inhibition**

| 1,5- Isoquinolinediol (300 µM) | | 32 hours | | 48 hours | |
|---|---|---|---|---|---|
| | | control | DIQ | control | DIQ |
| Control | Apoptosis | 2.24 | 8.15 | 16.05 | 20.16 |
| | Necrosis | 17.53 | 17.62 | 27.98 | 29.59 |
| GO | Apoptosis | 6.50 | 9.32 | 12.92 | 17.52 |
| | Necrosis | 27.11 | 16.69 | 31.87 | 20.89 |
| lin + GO | Apoptosis | 13.66 | 8.85 | 52.95 | 42.82 |
| | Necrosis | 32.91 | 23.23 | 25.07 | 30.06 |

## Claims

1. A system capable of causing the death of tumor cells by activating caspase-independent apoptosis, comprising: a cyanogenic system and an oxidative stress inducing system.

2. The system capable of causing the death of tumor cells by activating caspase-independent apoptosis according to claim 1, comprising:
a. a cyanogenic system and an oxidative stress inducing system combined in a single composition or
b. a cyanogenic system and an oxidative stress inducing system present in independent compositions.

3. The system according to claim 2, wherein said cyanogenic system comprises the linamarase-linamarin system.

4. The system according to claim 2, wherein said oxidative stress inducing system comprises the activity of the glucose oxidase enzyme.

5. The system according to claim 2, wherein said cyanogenic system comprises the linamarase-linamarin system and wherein said oxidative stress inducing system comprises the activity of the glucose oxidase enzyme.

6. The system according to claim 2, comprising the linamarase gene and the glucose oxidase gene combined in a single viral or non-viral vector.

7. The system according to claim 2, comprising the linamarase gene and the glucose oxidase gene in independent viral or non-viral vectors.

8. The composition according to claim 2.a, comprising the linamarase gene, introduced in a viral or non-viral vector, and the purified glucose oxidase protein or analogues, fragments or derivatives of said protein.

9. The composition according to claim 2.b, comprising the linamarase gene, introduced in a viral or non-viral vector, and the purified glucose oxidase protein or analogues, fragments or derivatives of said protein.

10. The composition according to claim 2.a, comprising the glucose oxidase gene, introduced in a viral or non-viral vector, and the purified linamarase protein or analogues, fragments or derivatives of said protein.

11. The composition according to claim 2.b, comprising the glucose oxidase gene, introduced in a viral or non-viral vector, and the purified linamarase protein or analogues, fragments or derivatives of said protein.

12. The composition according to claim 2.a, comprising the purified glucose oxidase protein and the purified linamarase protein or analogues, fragments or derivatives of said proteins.

13. The composition according to claim 2.b, comprising the purified glucose oxidase protein and the purified linamarase protein or analogues, fragments or derivatives of said proteins.

14. The composition according to any of claims 2-13 for its use in therapy.

15. Use of the composition according to any of claims 2-13 for preparing a pharmaceutical composition for treating tumors.

16. The pharmaceutical composition according to any of claims 14-15, and a pharmaceutically acceptable carrier.

17. The pharmaceutical composition according to any of claims 14-15, further comprising a controlled release system.

18. A pharmaceutical composition comprising a composition according to any of claims 8-13, wherein any of said proteins or both are bound to antibodies directed against tumor antigens.

19. The pharmaceutical composition according to claims 16-18, intended for treating breast cancer.

20. The pharmaceutical composition according to claims 16-18, intended for treating lung cancer.

21. The pharmaceutical composition according to claims 16-18, intended for treating head and neck cancer.

22. The pharmaceutical composition according to claims 16-18, intended for treating pancreatic cancer.

23. The pharmaceutical composition according to claims 16-18, intended for treating prostate cancer.

24. The pharmaceutical composition according to claims 16-18, intended for treating colon cancer.

25. The pharmaceutical composition according to claims 16-18, intended for treating melanomas.

26. The pharmaceutical composition according to claims 16-18, intended for treating osteosarcoma.

27. The pharmaceutical composition according to claims 16-18, intended for treating adenocarcinoma.

28. The pharmaceutical composition according to claims 16-18, intended for treating leukemia.

29. The pharmaceutical composition according to claims 16-18, intended for treating glioblastoma.

30. An in vitro method for causing the death of tumor cells by activating caspase-independent apoptosis, comprising the combination of the cyanogenic system and an oxidative stress inducing system.

31. The method according to claim 29, wherein the cyanogenic system comprises the linamarase-linamarin system.

32. The method according to claim 29, wherein the oxidative stress inducing system comprises the activity of the glucose oxidase enzyme.

33. The method according to claim 29, wherein the oxidative stress inducing system comprises the activity of the glucose oxidase enzyme and the cyanogenic system comprises the linamarase-linamarin system.

34. The method according to any of claims 29-32, wherein a vector/vectors are used comprising the linamarase and/or the glucose oxidase enzyme genes.

35. The method according to any of claims 29-32, wherein any of the purified linamarase and/or glucose oxidase proteins or derivatives, analogues or fragments of said proteins is used.

36. The method according to the previous claim, wherein any of said proteins or both proteins are inserted in a controlled release system.

37. The method according to any of claims 34-35, wherein any of said proteins or both proteins are bound to antibodies specific against tumor antigens.
